# EUROPÄISCHE PATENTSCHRIFT

(11) **EP 0 771 219 B1**
(45) Veröffentlichungstag und Bekanntmachung des Hinweises auf die Patenterteilung: **24.02.1999**
(21) Anmeldenummer: 96916077.9
(22) Anmeldetag: 14.05.1996
(51) Int. Cl.: A61M 5/30, A61M 37/00, A61B 17/22

(54) **EJEKTIONSGERÄT ZUR HOCHDRUCKEJEKTION EINER FLÜSSIGKEIT**
EJECTION APPARATUS FOR HIGH-PRESSURE EJECTION OF A LIQUID
APPAREIL D'EJECTION POUR L'EJECTION HAUTE PRESSION D'UN LIQUIDE

(30) Priorität: 15.05.1995 DE 29507987 U
(43) Veröffentlichungstag der Anmeldung: 07.05.1997
(73) Patentinhaber: Ferton Holding, CH-2800 Delemont (CH)
(72) Erfinder: MENNE, Andreas, D-88709 Meersburg (DE); MERKLE, Wolfgang, D-52441 Linnich (DE); SCHULZ, Manfred, D-88662 Überlingen (DE); KLOPFENSTEIN, Denis, CH-1110 Morges (CH)
(74) Vertreter: Viering, Jentschura & Partner
(86) Internationale Anmeldenummer: EP9602072
(87) Internationale Veröffentlichungsnummer: WO9636381

(56) Entgegenhaltungen:
- EP-A- 0 317 507
- DE-A- 3 812 841
- US-A- 3 490 696
- US-A- 5 116 313

## Beschreibung

Die Erfindung betrifft ein Ejektionsgerät zur Hochdruckejektion einer Flüssigkeit, oder einer Feststoffpartikel enthaltenden Flüssigkeit, mit einer die Flüssigkeit aufnehmenden Druckkammer, die in eine Ejektionsöffnung ausmündet und die von einem Arbeitskolben begrenzt wird, der von einem Antrieb in der Druckkammer verlagerbar ist, welcher als Anschlagstück ausgebildet ist, das angetrieben bis zum Anschlagen an dem der Druckkammer abgewandten Ende des Arbeitskolbens beschleunigbar ist, wobei das Volumen der Druckkammer größer ist als das Verdrängungsvolumen der von dem Arbeitskolben bei dessen Arbeitshub verdrängten Flüssigkeit.

Ein derartiges Ejektionsgerät zur Hochdruckejektion einer Flüssigkeit ist aus der US-A-3 490 696 bekannt, wobei das Anschlagstück als zu dem Arbeitskolben koaxialer Antriebskolben ausgebildet ist, der in einem Antriebsrohr periodisch hin- und hergehend z.B. pneumatisch oder elektrisch angetrieben wird. Nach dem Anschlagen des Anschlagstücks an dem Arbeitskolben bewegen sich diese gemeinsam gegen den Widerstand der in der Druckkammer und durch die Ejektionsöffnung verdrängten Flüssigkeit, bis ihre kinetische Energie aufgezehrt und an den ausgegebenen Flüssigkeitsstrahl übergeben ist. Danach wird der Arbeitskolben mithilfe einer Feder zurückgestellt und ist damit für einen neuen Ejektionszyklus bereit. Dieses bekannte Ejektionsgerät ist insbesondere zur Bearbeitung von Gestein, Metall, Beton, Holz, Stoff oder dergl. vorgesehen.

Bei bekannten nadellosen Impfgeräten wird der Arbeitskolben zumeist von einer gespannten Feder angetrieben, wobei der Arbeitskolben mit verhältnismäßig hohem Druck auf die Flüssigkeit einwirkt und das gesamte in der Druckkammer vorhandene Flüssigkeitsvolumen aus der Ejektionsöffnung ausgestoßen wird. Es ist auch bekannt (z.B. GB-PS 993 309), die Druckkammer als Ampulle mit verformbaren Ampullenwänden auszubilden und als Antrieb einen Antriebskolben zu verwenden, der zunächst einen Stoßimpuls auf den Arbeitskolben ausübt, um anfänglich einen höheren Ejektionsdruck für die Durchdringung der Haut zu erreichen, wonach der Arbeitskolben von einer Federanordnung nachgeschoben wird, um das restliche Flüssigkeitsvolumen aus der Ampulle auszudrücken.

Bei diesem Flüssigkeitsvolumen handelt es sich im allgemeinen um relativ große Mengen im Bereich von Kubikzentimetern, was zu einer traumatischen Wirkung auf das die Impfstelle umgebende Gewebe führen kann. Andererseits besteht Bedarf dafür, ggf. kleine Flüssigkeitsmengen zu injizieren. Es wurde nämlich festgestellt, daß bei einer Hochdruckinjektion von kleinsten Farbstoffmengen in Gewebe der Farbstoff bei einer deutlich geringeren Schädigung des Gewebes gleichmäßig verteilt wurde.

Durch die Erfindung wird das Problem gelöst, wie ein Ejektionsgerät der eingangs erwähnten Art so ausgebildet werden kann, daß auch kleine Flüssigkeitsmengen, ggf. Kubikmillimeter, ejiziert werden können.

Dies wird erfindungsgemäß dadurch erreicht, daß von dem Anschlagstück ein elastischer Stoß auf den Arbeitskolben ausübbar ist und das Anschlagstück mit der Stoßübertragung antriebslos ist.

Durch das Anschlagen des Anschlagstücks auf den Arbeitskolben wird auf diesen ein elastischer Stoß übertragen, wodurch der Arbeitskolben einen Hochdruckimpuls auf die Flüssigkeit in der Druckkammer überträgt, der zur Hochdruckejektion der Flüssigkeit aus der Ejektionsöffnung führt. Hierbei sind der Ejektionsdruck und auch das ejizierte Volumen der Flüssigkeit abhängig von der Auftreffgeschwindigkeit des Anschlagstücks auf den Arbeitskolben und können daher durch Einstellung der Antriebsgeschwindigkeit des Anschlagstücks eingestellt werden. Da das Antriebsstück mit der Stoßübertragung seine Antriebskraft verliert, wird auf die Flüssigkeit nur der kurze Stoßimpuls übertragen. Durch den kurzen Hochdruckimpuls, der auf die Flüssigkeit in der Druckkammer übertragen wird, wird eine entsprechend kleine Teilmenge der Flüssigkeit aus der Ejektionsöffnung ejiziert, ohne das Gesamtvolumen der Druckkammer zu entleeren. Anders als im Stand der Technik wird die Stoßübertragung nur ausgenutzt, diese kleine Teilmenge zu ejizieren, ohne anschließend das Gesamtvolumen der Druckkammer auszustoßen. Dies wird dadurch erreicht, daß das Anschlagstück einen elastischen Stoß auf den Arbeitskolben ausübt und mit der Stoßübertragung, d.h. mit Abgabe des Stoßimpulses, antriebslos wird und keine weitere Antriebskraft auf den Arbeitskolben ausübt. Es ist dadurch möglich, eine nur kleine Menge der Flüssigkeit dosiert auszugeben.

Das Druckkammervolumen ist größer als das Hubvolumen des Arbeitskolbens, um unabhängig von der Größe der Druckkammer eine kleine, aber dosierte Menge der Flüssigkeit ejizieren zu können. Vorzugsweise beträgt das Verhältnis des Druckkammervolumens zu dem Verdrängungsvolumen des Antriebskolbens 5:1 bis insbesondere 20:1, das durch Einstellung der Antriebsgeschwindigkeit des Anschlagstücks, d.h. der Größe des auf den freibewegbaren Antriebskolben übertragenen Stoßimpulses, veränderbar sein kann.

Die Antriebslosigkeit des Anschlagstücks nach der Stoßimpulsübertragung auf das Anschlagstück kann beispielsweise mithilfe eines Anschlags erreicht werden, von dem das Anschlagstück nach der Stoßimpulsübertragung abgestoppt wird, oder z.B. durch entsprechende zeitliche Begrenzung des auf das Anschlagstück einwirkenden Antriebsimpulses.

Der Arbeitskolben besteht insbesondere aus einem festen, vorzugsweise aber elastisch biegbaren Material, in dem elastische Stoßwellen übertragen werden, wie z.B. einem Metallmaterial. Bevorzugt wird als solches Metallmaterial Stahl oder Titan oder eine Titanlegierung. Das Anschlagstück kann irgendein Bauteil sein, das angetrieben auf den Arbeitskolben aufschlägt, beispielsweise ein Kipphebel, der von einem Antriebskolben angetrieben wird, oder z.B. eine schwenkbare Klappe, deren Ende auf den Arbeitskolben aufschlägt. Bevorzugt ist das Anschlagstück als insbesondere koaxial zu dem Arbeitskolben ausgerichteter Antriebskolben ausgebildet, der in einem Antriebsrohr angetrieben beschleunigbar ist.

Das Anschlagstück kann insbesondere pneumatisch, hydraulisch, mechanisch, elektromagnetisch, elektrostriktiv, piezoelektrisch oder thermisch angetrieben sein. Hierbei ist es möglich, den Antrieb des Anschlagstücks so zu gestalten, daß dieser für jeweils nur einen Antriebshub angetrieben wird. Bevorzugt wird es jedoch, wenn das Anschlagstück für ein gesteuert mehrfaches Anschlagen an dem Arbeitskolben in periodischer Wiederholung antreibbar ist, wobei das Anschlagstück und der Arbeitskolben selbsttätig rückstellbar sind. Hierdurch kann die Gesamtejektionsmenge in Abhängigkeit von der Anzahl der Wiederholungen gesteuert eingestellt werden.

Die zu ejizierende Flüssigkeit kann auch mit kleinen Feststoffpartikeln versetzt sein. Medikamente können z.B. zum Zwecke der besseren Dosierung in Trägerpartikel eingebettet werden, die dann im Körper wohldosiert das Medikament freisetzen. Sind solche oder auch andere Partikel der Flüssigkeit in der Druckkammer zugesetzt, so werden diese bei dem Ejektionsvorgang mit ausgebracht und verhalten sich vergleichbar mit der sie umgebenden Flüssigkeit. Daher kann mit dem Ejektionsgerät ein Medikamentendepot im Gewebe oder auch eine Teilchenanlagerung zu anderen Zwecken angelegt werden.

Die Ejektionsöffnung kann, wenn sie klein genug ist, offen sein. Hierbei kann die Ejektionsöffnung als sich verengende Düse oder als Kanal in ihrem Volumen derart ausgebildet sein, daß die nach einer Ejektion ggf. zurückgesaugte Flüssigkeit oder Luft innerhalb der Düse verbleibt und nicht in die Druckkammer eindringt. Durch das Nachfüllen der Druckkammer mit frischer Flüssigkeit wird somit zuerst die sich am Auslaßende der Düse befindende Substanz verdrängt, so daß bei einem nachfolgenden Hochdruckimpuls keine zurückgesaugten Substanzen ejiziert werden.

Es ist aber auch möglich, daß die Ejektionsöffnung von einer Ventilvorrichtung beherrscht ist, die gesteuert angetrieben ist oder - vorzugsweise - auf eine vorbestimmte Drucksteigerung in der Druckkammer unter Öffnen der Ejektionsöffnung anspricht und druckabhängig rückstellbar ist. Hierdurch wird insbesondere verhindert, daß beim Abfallen des Druckes in der Druckkammer nach der Flüssigkeitsejektion unter den Umgebungsdruck Luft oder Flüssigkeit in die Druckkammer zurückgesaugt wird. Die Ventilvorrichtung kann beispielsweise ein federbelasteter Ventilstößel oder Ventilschieber sein. Es ist insbesondere aber auch möglich, die Ejektionsöffnung in einer die Druckkammer begrenzenden elastischen Membran auszubilden und in der Ruhelage der Membran vom freien Ende eines Verschlußstiftes verschlossen zu halten. Unter dem Ejektionsdruck in der Druckkammer wird dann die Membran elastisch ausgewölbt, so daß die Ejektionsöffnung vom freien Ende des Verschlußstiftes abgehoben und damit geöffnet wird und mit dem Absinken des Druckes wieder in die Ruhelage zurückgestellt wird, in der die Ejektionsöffnung wieder verschlossen wird.

Für einen periodischen Betrieb des Gerätes weist die Druckkammer ein durch eine Druckabnahme in der Druckkammer öffnendes Einlaßventil auf, so daß die Druckkammer durch das Absinken des Flüssigkeitsdruckes wieder nachgefüllt wird.

Weiter kann die Druckkammer in ihrem Aufnahmevolumen verstellbar sein. Dadurch lassen sich der Ejektionsdruck und die Ejektionsdauer einstellen, denn bei einer Erhöhung des Druckkammervolumens werden der Ejektionsdruck verringert und die Ejektionsdauer verlängert.

Eine vorteilhafte Ausführungsform der Erfindung ist gekennzeichnet durch die Ausbildung als endoskopisches Gerät, wobei der Arbeitskolben als elastische Stoßwellen auf die Flüssigkeit in der Druckkammer übertragende lange elastisch biegbare Sonde ausgebildet ist. Aus der EP 0 317 507 ist ein endoskopischer Ultraschall-Generator bekannt, bei welchem ein Antriebskolben periodisch gegen eine elastische Stoßwellen übertragende Sonde anschlägt, mit der dadurch Nierensteine zertrümmert werden können.

Der Arbeitskolben kann aber bei der Ausführungsform der Erfindung als endoskopisches Gerät auch kurz ausgeführt sein und die Druckkammer als langgestreckter flüssigkeitsgefüllter Kanal in dem elastischen Endoskop-Katheter ausgebildet sein. Hierbei wird die Stoßwelle durch die im Kanal befindliche Flüssigkeitssäule übertragen, wodurch der Endoskopkatheter weitergehend flexibel ausgebidet sein kann.

Bei dem erfindungsgemäßen Ejektionsgerät kann die Druckkammer mit einem Zulaufkanal von einem Flüssigkeitsvorrat verbunden sein, in dem beispielsweise ein zu dem Flüssigkeitsvorrat hin sperrendes Rückschlagventil angeordnet ist. Die Druckkammer kann aber auch über einen von einem Flüssigkeitsvorrat kommenden engen Zulaufkanal verbunden sein, der eng genug ist, um die Druckerhöhung in der Druckkammer während des Auftretens des zur Ejektion führenden Hochdruckimpulses nicht wesentlich zu schmälern. Beispielsweise kann bei einer Ausführungsform des erfindungsgemäßen Hochdruckejektionsgerätes als endoskopisches Gerät die Sonde an ihrem der Druckkammer benachbarten Endabschnitt in einem Führungsstück gleitend geführt sein, zwischen dem und der Sonde ein enger Flüssigkeitsdurchtrittsspalt ausgebildet ist, der proximal in einen entlang der Sonde verlaufenden Flüssigkeitszuführkanal mündet. Hierbei bildet der enge Flüssigkeitsdurchtrittsspalt das Einlaßventil, weil die Flüssigkeit nur dann durch den Flüssigkeitsdurchtrittsspalt in die Druckkammer nachfließen kann, wenn dort sich der Druck wieder verringert hat, wohingegen der kurze Hochdruckimpuls keinen nennenswerten Flüssigkeitstransport durch den engen Durchtrittsspalt bewirken kann. Ebenso eignet sich jede kleine Flüssigkeitsdurchtrittsöffnung zur Druckkammer als Flüssigkeitszufuhr.

Im allgemeinen ist nur eine Ejektionsöffnung vorgesehen, in welche die Druckkammer ausmündet. Insbesondere bei einer Ausführungsform der Erfindung als endoskopisches Gerät kann es jedoch vorteilhaft sein, mehrere Ejektionsöffnungen vorzusehen, um beispielsweise ein größeres Gewebegebiet mit ejiziertem Impfstoff zu versorgen.

Die Erfindung wird anhand von Ausführungsformen erläutert, die aus der Zeichnung wenigstens schematisch ersichtlich sind. In der Zeichnung zeigt:
Fig. 1 ein bevorzugtes Ausführungsbeispiel des Antriebsteils eines erfindungsgemäßen Ejektionsgerätes,
Fig. 2 bis 5 vier Ausführungsformen des Ejektionsteils eines erfindungsgemäßen Ejektionsgerätes, ausgeführt als endoskopisches Ejektionsgerät, welches mit dem Antriebsteil aus Fig. 1 zusammenwirken kann, und
Fig. 6 eine Ausführungsform eines Hochgeschwindigkeitsinjektors, der ebenfalls mit dem Antriebsteil aus Fig. 1 zusammenwirken kann.

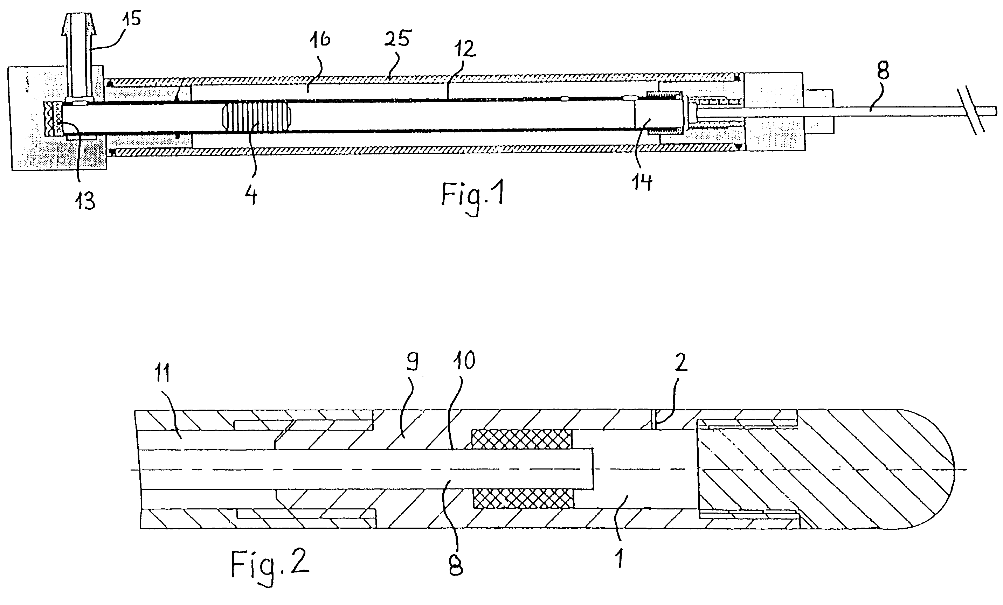

In Fig. 1 ist ein Ausführungsbeispiel des Antriebsteils eines erfindungsgemäßen Ejektionsgerätes zur Hochdruckinjektion einer Flüssigkeit dargestellt. In einem Beschleunigungsrohr 12 ist ein Antriebskolben 4 zwischen einem Ausgangsanschlag 13 an dem einen Rohrende und einem Sondenkopf 14 eines als Sonde 8 ausgebildeten Arbeitskolbens am anderen Rohrende hin und her verschiebbar. Im Ausführungsbeispiel wird der Antriebskolben 4 pneumatisch angetrieben, wobei die Druckluft durch den Druckluftstutzen 15 zugeführt wird. Rings des Beschleunigungsrohrs 12 ist eine Ringkammer 16 ausgebildet, die nahe des Sondenkopfes 14 mit dem Inneren des Beschleunigungsrohres 12 in Verbindung steht. Durch Zufuhr eines Druckluftimpulses in das Beschleunigungsrohr 12 wird der Antriebskolben 4 zu dem anderen Rohrende getrieben, bis er an dem Sondenkopf 14 anschlägt. Die Luft im Beschleunigungsrohr stromabwärts des Antriebskolbens wird in die Ringkammer 16 verdrängt und dort verdichtet. Nach dem Zuführen der Druckluft wird der Antriebskolben 4 in dem Beschleunigungsrohr 12 durch den in der Ringkammer 16 aufgebauten Druck bis zum Anschlagen an dem Ausgangsanschlag 13 zurückgeführt.

Für weitere Einzelheiten des Antriebsteils aus Fig. 1 wird auf die diesbezügliche Offenbarung in der EP 0 317 507 A1 Bezug genommen, in der ein solches Antriebsteil im Zusammenhang mit einem endoskopischen Nierensteinzertrümmerer mithilfe der Sonde 8 beschrieben ist.

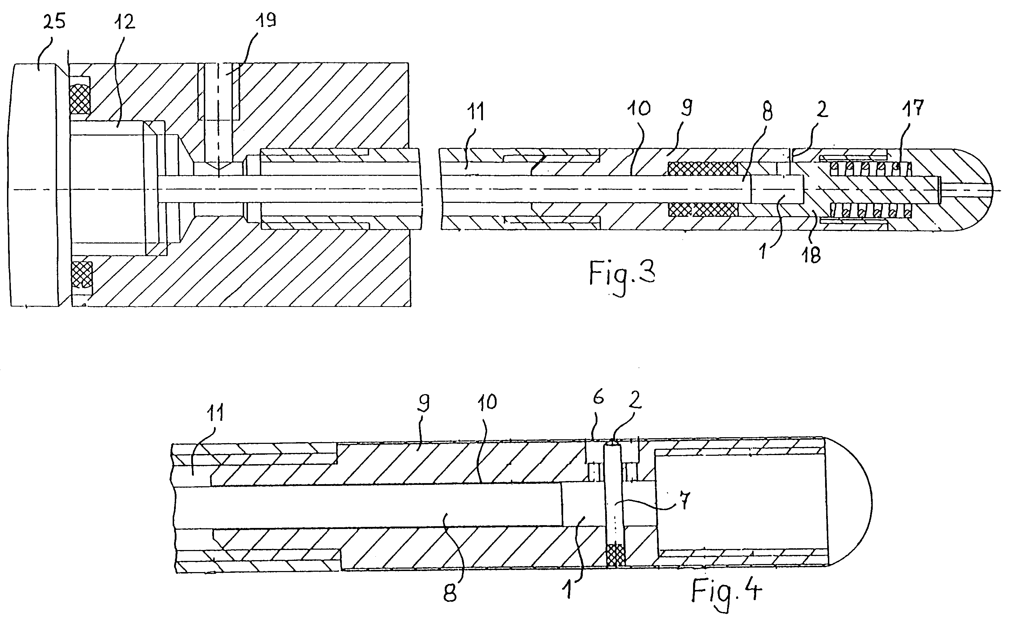

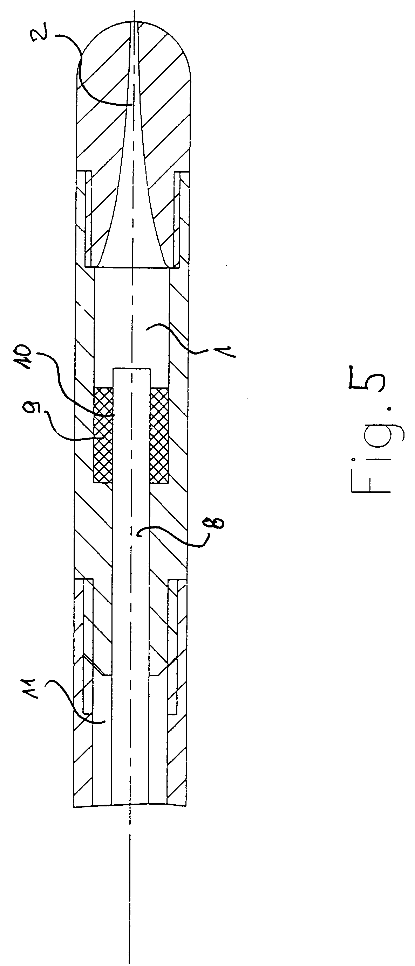

Erfindungsgemäß dient die langgestreckte Sonde 8 als Arbeitskolben für die Hochdruckejektion einer Flüssigkeit. Ausführungsformen für ein erfindungsgemäßes endoskopisches Injektionsgerät sind aus den Figuren 2 bis 5 ersichtlich. Von dem distalen Ende der Sonde 8 wird eine die Flüssigkeit enthaltende Druckkammer 1 begrenzt, die in eine Ejektionsöffnung 2 ausmündet. Nach Fig. 2 ist die enge Ejektionsöffnung 2 ständig offen; nach Fig. 3 wird die Ejektionsöffnung 2 von einer Ventilvorrichtung in Form eines gegen die Rückstellkraft einer Feder 17 verschiebbaren Ventilschiebers 18 beherrscht. Nach Fig. 4 wird die Druckkammer 1 von einer Membran 6 umgrenzt, in der die Ejektionsöffnung 2 ausgebildet ist, die jedoch in der Ruhelage vom freien Ende eines Verschlußstiftes 7 verschlossen wird.

Der distale Endabschnitt der Sonde 8 ist in einem Führungsstück 9 gleitend derart geführt, daß zwischen der Sonde und dem Führungsstück ein enger Flüssigkeitsdurchtrittsspalt 10 verbleibt, der proximal in einen die Sonde 8 umgebenden Flüssigkeitszuführkanal 11 mündet. Die Flüssigkeitszufuhr erfolgt am Eintrittsstutzen 19.

Das in den Fig. 2 bis 4 dargestellte endoskopische Hochgeschwindigkeits-Injektionsgerät kann dort Verwendung finden, wo Flüssigkeiten an schwierig zugänglichen Orten nadellos injiziert bzw. appliziert werden sollen. Der Hauptanwendungsbereich kann in der Medizin bei minimalinvasiven Operationstechniken zu sehen sein. Dabei ist insbesondere an die medikamentöse Behandlung der Prostata mit o-Blockern gedacht. Weitere denkbare Anwendungsgebiete sind eine medikamentöse Tumorbehandlung oder auch eine Injektion von Substanzen durch die Zellmembran hindurch für eine Gentherapie. Jedoch gibt es auch eine Vielzahl anderer Einsatzmöglichkeiten.

Das endoskopische Ejektionsgerät ist vergleichbar mit einer miniaturisierten Impfpistole, deren kompressionserzeugender Mechanismus weit von der Ejektionsöffnung 2 entfernt ist. Beispielsweise können der Durchmesser des Führungsstückes 9 etwa 6 mm, und der Durchmesser der Sonde 8 etwa 2 bis 3,5 mm betragen. Das Flüssigkeitsvolumen in der Druckkammer 1 wird durch die elastische Auslenkung der Sonde 8 unter Druck gesetzt. Dabei entstehen kurzzeitig sehr hohe Drücke (etwa 50 MPa), die zu einem Flüssigkeitsausstoß durch die Ejektionsöffnung führen.

Die Druckenergie wird durch den im Ausführungsbeispiel pneumatisch beschleunigten Antriebskolben 4 (Fig. 1) bereitgestellt, der am Ende des Beschleunigungsrohres 12 auf den Sondenkopf 14 aufschlägt. Dabei wird eine Kompressionswelle in der Sonde 8 erzeugt, die sich entlang dieser fortbewegt. Erreicht die Kompressionswelle das Ende der Sonde 8, so wird durch deren Vorwärtsbewegung das Flüssigkeitsvolumen in der Druckkammer 1 verkleinert. Dies führt zu einem Druckanstieg in der Flüssigkeit. Aus der in der Druckkammer 1 seitlich angebrachten Ejektionsöffnung 2 spritzt nun infolge der Druckerhöhung Flüssigkeit mit hoher Geschwindigkeit.

Der Flüssigkeitszuführkanal 11 erlaubt einen kontinuierlichen Betrieb des Ejektors. Am Eintrittsstutzen 19 wird die Flüssigkeit mit geringem Druck zugeführt (z.B. hydrostatisch). Zwischen Sonde 8 und Führungsstück 9 kann die Flüssigkeit über den engen Durchtrittsspalt 10 in die Druckkammer 1 gelangen.

Die Zufuhr der Flüssigkeit in die Druckkammer 1 muß durch ein mechanisches oder hydraulisches Einlaßventil geregelt sein, das einerseits zwischen den Kompressionsvorgängen Flüssigkeit in die Druckkammer läßt, aber andererseits während des Kompressionsvorganges schließt oder wesentlich sperrt, um den Druck überhaupt aufbauen zu können. Dies ist in den gezeigten Ausführungsformen durch den langen Durchtrittsspalt 10 zwischen Sonde 8 und Führungsstück 9 realisiert, der durch die unterschiedlich lang andauernden Vorgänge des unter Drucksetzens und der Nachfüllung die Funktion eines Ventils übernimmt. Das Nachfüllen der Druckkammer 1 kann immer dann stattfinden, wenn der Druck in dieser nicht erhöht ist. Bei geeigneten Abmessungen von Düsenöffnung und Druckkammer dauert die Drucküberhöhung 50 µs bis 1 ms. Bei einer Schußfrequenz, die durch den Beschleunigungsvorgang des Antriebskolbens 4 beschränkt ist, (z.B. 20 Hz), bleibt folglich eine relativ lange Zeit, um genügend Flüssigkeit bei kleiner Druckdifferenz auch durch einen sehr engen Durchtrittspalt 10 zu transportieren. Während der sehr kurz andauernden Kompression kann hingegen durch den langen engen Durchtrittsspalt nur eine minimale Flüssigkeitsmenge ausströmen, wodurch die maximal erreichbare Druckerhöhung kaum gemindert wird. Der Spalt wirkt als instationäre Dichtung.

Nach der Druckerhöhung in der Druckkammer 1 sinkt der Druck kurzzeitig unter den Umgebungsdruck. Dies führt bei einer Anwendung, bei der die Ejektionsöffnung 2 nicht mit Flüssigkeit umgeben ist, zu einem Ansaugen der Umgebungsluft. Kann diese eingesaugte Luft nicht durch die nachgelieferte Flüssigkeit wieder aus der Druckkammer 1 verdrängt werden, bevor der nächste Kompressionsvorgang beginnt, so muß das Instrument entlüftet werden. Daher ist bevorzugt eine Ventilvorrichtung vorgesehen, wodurch das Ejektionsgerät sowohl in Gas als auch in Flüssigkeit eingesetzt werden kann. Fig. 3 zeigt einen durch den Kompressionsdruck verschiebbaren Ventilschieber 18, der die Ejektionsöffnung 2 freigibt oder schließt. Ein weiterer Vorschlag ist in Fig. 4 dargestellt. Um die bewegten Massen für die sehr kurzen Steuerzeiten so gering wie möglich zu halten, ist die Wand der Druckkammer 1 als elastische Membran 6 ausgeführt, in der sich gleichzeitig auch die Ejektionsöffung 2 befindet. Die Ejektionsöffnung 2 wird von dem freien Ende eines Stiftes 7 verschlossen. Durch die Druckerhöhung in der Druckkammer 1 wird die elastische Membran 6 von dem freien Ende des Verschlußstiftes 7 abgehoben und gibt einen kleinen Spalt frei. Durch diesen kann nun die Flüssigkeit in die Ejektionsöffnung 2 strömen und nach außen gelangen.

Die ausgespritzte Flüssigkeitsmenge pro Schuß liegt aufgrund der kurzen Kompressionszeiten der elastischen Welle unterhalb von 5 µl. Herkömmliche Impfpistolen bringen das 300 bis 1000-fache Flüssigkeitsvolumen aus.

Das endoskopische Ejektionsgerät gemäß der Erfindung weist insbesondere folgende Vorteile auf:
- sehr gute Dosierbarkeit des Injektionsvolumens durch minimale Ausbringungsmengen je Schuß;
- gleichmäßige Verteilung auch geringster Volumina im Gewebe;
- hohe Eindringtiefe der zu injizierenden Flüssigkeit;
- minimale Verletzung des Gewebes;
- hohe Schußfolge der einzelnen Injektionen durch automatischen Nachfüllmechanismus (zur Zeit sind 20 Schuß pro Sekunde möglich);
- sehr kurze steile Kompression durch Impulsübertragung.

Während nach den Figuren 2 bis 4 die Ejektionsöffnung 2 seitlich des Sondenkopfes ausgebildet ist, zeigt Fig. 5 eine Ausführungsform, bei der die Ejektionsöffnung 2 stirnseitig der Sonde ausmündet. In der Ausführungsform aus Fig. 5 ist der Ejektionskanal als sich trichterförmig verjüngende Düse ausgestaltet.

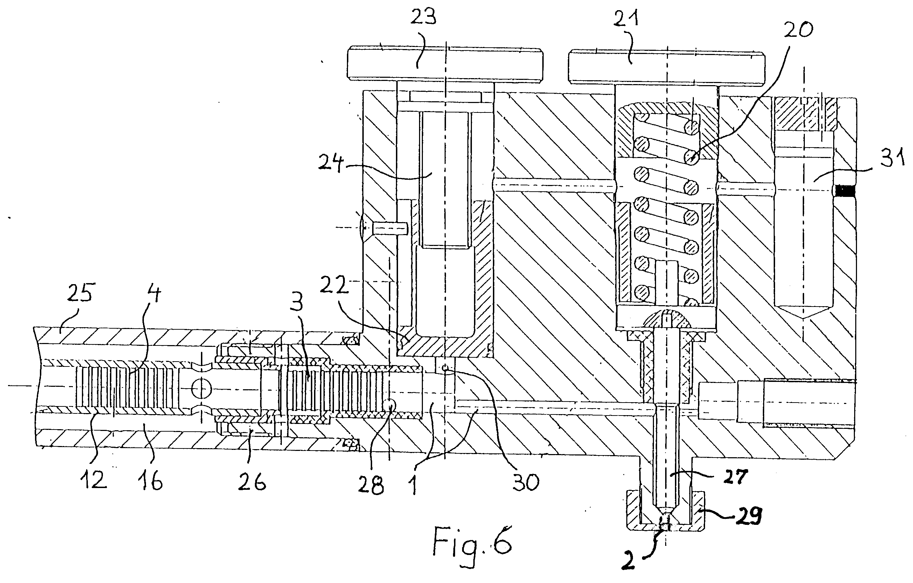

Aus Fig. 6 ist eine weitere Ausführungsform der Erfindung in Form eines nadellosen Impfgerätes ersichtlich. Hier ist anstelle der langen Sonde 8 bei den Ausführungsformen nach den Fig. 2 bis 5 ein kurzer Arbeitskolben 3 vorgesehen. Die Ejektionsöffnung 2 wird von einem Nadelventil beherrscht, dessen Ventilfeder 20 in deren Federkraft mittels eines Handrades 21 verstellbar ist. Außerdem wird die Druckkammer 1 von einem Verstellkolben 22 begrenzt, der mittels eines zweiten Handrades 23 mit Verstellspindel 24 zur Änderung des Aufnahmevolumens der Druckkammer 1 verstellbar ist.

Als Antriebsteil kann derjenige nach Fig. 1 dienen, dessen Außenrohr 25 statt an die Sondenführung an den den Arbeitskolben 3 aufnehmenden Gerätestutzen 26 angeschraubt wird, so daß das Beschleunigungsrohr 12 auf den Arbeitskolben 3 ausgerichtet ist.

Auch bei der Ausführungsform aus Fig. 6 wird der Antriebskolben 4 im Beschleunigungsrohr 12 pneumatisch beschleunigt, bis der Antriebskolben 4 auf den Arbeitskolben 3 auftrifft (1. Stoß). Da dieser Stoß nicht völlig elastisch ist, wird nur ein Teil (nahe bei 100%) der kinetischen Energie auf den Arbeitskolben 3 übertragen. Beim Vorliegen eines vollständig elastischen Stoßes würde der Antriebskolben 4 nach dem Stoß stehenbleiben und der Arbeitskolben 3 würde mit der Geschwindigkeit des Antriebskolbens 4 weiterfliegen (Es wird hierzu vorausgesetzt, daß die Kolben gleiche Masse und Länge haben). Da aber ein kleiner Teil der Energie beim Stoß verlorengeht, fliegen beide Kolben 3, 4 in Richtung zu der Ventilnadel 27 weiter, der Antriebskolben 4 jedoch mit einer sehr viel kleineren Geschwindigkeit. Die Beschleunigung des Arbeitskolbens 3 erfolgt in der Zeit, die die elastischen Wellen im Material der Kolben brauchen, um einmal hin- und zurückzulaufen. Diese Zeit ist kurz (bei Kolben von 20 mm Länge ca. 8 µs) im Vergleich zur Einspritzdauer. Auch die akustischen Wellenlaufzeiten in dem unter Druck zu setzenden Flüssigkeitsvolumen sind kürzer als die Einspritzdauer (bei einer max. Kolbenlänge von 15 mm und Wasser als Flüssigkeit: 10 µs). Deshalb wird das Flüssigkeitsvolumen durch die Bewegung des Arbeitskolbens 3 als ganzes gleichmäßig unter Druck gesetzt. Die Druckerhöhung bewirkt, daß der Arbeitskolben 3 stetig abgebremst wird und später die Bewegungsrichtung umkehrt. Die kinetische Energie wird in Druckenergie umgewandelt und umgekehrt.

Nach der Ejektion der Flüssigkeit stößt der Arbeitskolben 3 zum zweitenmal mit dem Antriebskolben 4 zusammen. Letzterer fliegt dann in dem Beschleunigungsrohr 12 zurück. Der Arbeitskolben 3 behält eine kleine Geschwindigkeit, die ausreichend ist, daß er in die Ruhelage zurückkehrt. Hierbei öffnet das druckgesteuerte Einlaßventil (nicht gezeigt), das in einer in der Ruhelage des Arbeitskolbens 3 von diesem freigegebenen Einlaßöffnung 28 mündet, weil der Druck in der Druckkammer 1 nun geringer als der Druck in einem Vorratsbehälter (z.B. Spritze) ist. Die ausgespritzte Masse wird hierdurch nachgefüllt.

Die Druckerhöhung in der Druckkammer 1 bewirkt eine Zunahme der Kraft auf die Schulter der Ventilnadel 27. Sobald diese Druckkraft die Kraft der vorgespannten Ventilfeder 20 übersteigt, hebt die Ventilnadel 27 ab und der Ejektionsvorgang beginnt. Durch die Bewegungsumkehr des Arbeitskolbens 3 erreicht der Druck ein Maximum, um dann abzufallen. Fällt der Druck unter den Öffnungsdruck, so wird die Ejektionsöffnung 2 von der Ventilnadel 27 wieder geschlossen und der Ejektionsvorgang wird beendet. Unter dem noch verbleibenden Flüssigkeitsdruck wird der Arbeitskolben 3 in die Ruhelage geschoben.

Der Öffnungsdruck wird über die Vorspannung der Ventilfeder 20 eingestellt. Eine kleine Steigung des Gewindes (0,5 mm) der Stellschraube 21 erlaubt die Einstellung des Öffungsdruckes zwischen 10 und 200 bar bei einer Umdrehung. Es mag so scheinen, daß die Feder (Federkonstante z.B. 200 N/mm) sehr groß und steif ist. Dies ist aber deshalb vorteilhaft, damit die Nadel schnell genug schließt. Eine weiche Feder könnte zwar durch stärkere Kompression die gleiche Kraft aufbringen, aber da es sich um ein Masse-Feder System handelt, muß dessen Resonanzfrequenz hoch genug liegen, damit der Schließvorgang beendet ist, ehe der Druck im System unter den Umgebungsdruck fällt. Andernfalls würde durch die Ejektionsöffnung 2 Luft angesaugt werden.

Die auf den Ausgangsstutzen aufgeschraubte Kappe 29 mit der Ejektionsöffnung 2 ist als separates Teil vorgesehen, um zum einen die Herstellung zu erleichtern, aber zum zweiten, um Ejektionsöffnungen 2 unterschiedlicher Größe benutzen zu können. Die Größe der Ejektionsöffnung entscheidet in erheblichem Maße darüber, wieviel Flüssigkeit ejiziert wird. Eine Vergrößerung auf den zweifachen Wert würde das Ejektionsvolumen vervierfachen, ohne einen nennenswerten Einfluß auf den Ejektionsdruck zu haben. Da aber diese Möglichkeit einen Umbau beinhaltet, ist die Variation des Volumens der Druckkammer 1 vorgesehen, wodurch das Ejektionsvolumen wenn auch in geringerem Maße, aber ohne Umbau, verändert werden kann.

Zum Entlüften und Befüllen der Druckkammer 1 wird der Verstellkolben zur Verstellung des Volumens der Druckkammer 1 auf deren minimales Volumen eingestellt. Knapp unterhalb der Kolbenkante befindet sich die Entlüftungsöffnung 30, die im Betriebszustand des Gerätes mit einer Schraube (nicht gezeigt) verschlossen ist. Diese Schraube wird leicht gelockert. Mit einer Füllspritze an der Einlaßöffnung 28 wird die Flüssigkeit in die Druckkammer 1 gedrückt. Hierbei öffnet das Einlaßventil, das auf Überdruck von außen (Unterdruck im Inneren) öffnet. Es wird solange Flüssigkeit nachgedrückt, bis diese an der Entlüftungsöffnung 30 herausquillt. Dann wird die Schraube an der Entlüftungsöffnung 30 dichtgezogen. Durch Drehen an der Verstellspindel 24 kann jetzt das gewünschte Gesamtvolumen nachgefüllt werden, denn durch das Herausziehen des Verstellkolbens entsteht ein Unterdruck und das Einlaßventil öffnet, so daß dann Flüssigkeit aus der Vorratsspritze herausgezogen wird. Für die Vorratsspritze sollte deshalb eine Glasausführung benutzt werden, die leichtgängig ist.

Da stets kleine Leckagen an der Ventilnadel 27 wie auch an dem Verstellkolben 22 zu erwarten sind, ist ein Leckagesammelsystem 31 vorgesehen.

Rechts in Fig. 6 ist eine Druckwandlerbohrung vorgesehen, die zwar für den Gerätebetrieb nicht notwendig ist, jedoch für Funktionstests und für die Eichung der Stellschrauben vorteilhaft ist. Bei normalem Betrieb soll daher hier ein Blindbolzen die Öffnung abschließen.

Wie bereits erwähnt, kann mithilfe des Verstellkolbens 22 das Volumen der Druckkammer 1 verändert werden. Hierdurch und durch die Veränderung der Geschwindigkeit des Antriebskolbens 4 können sowohl der Ejektionsdruck als auch das Ejektionsvolumen verändert werden. Eine Erhöhung der Geschwindigkeit des Antriebskolbens 4 durch eine Erhöhung des pneumatischen Antriebsdrucks bewirkt sowohl eine Erhöhung des Ejektionsdruckes als auch des Ejektionsvolumens. Im wesentlichen ist das Maximum des Ejektionsdruckverlaufes direkt proportional zur Kolbengeschwindigkeit. Das Ejektionsvolumen nimmt in erster Näherung mit der Wurzel der Kolbengeschwindigkeit zu. Durch Verringerung des Volumens der Druckkammer 1 und Erhöhung der Geschwindigkeit des Antriebskolbens kann daher der Ejektionsdruck erhöht und die mit der Erhöhung der Kolbengeschwindigkeit ebenfalls einhergehende Vergrößerung des Ejektionsvolumens wenigstens teilweise kompensiert werden. Wird das Volumen der unter Druck gesetzten Flüssigkeit bei gleichbleibender Kolbengeschwindigkeit vergrößert, so erniedrigt sich der Ejektionsdruck und die Ejektionsdauer wird verlängert

## Patentansprüche

1. Ejektionsgerät zur Hochdruckejektion einer Flüssigkeit, oder einer Partikel enthaltenden Flüssigkeit, mit einer die Flüssigkeit aufnehmenden Druckkammer (1), die in eine Ejektionsöffnung (2) ausmündet und die von einem Arbeitskolben (3, 8) begrenzt wird, der von einem Antrieb in der Druckkammer (1) verlagerbar ist, welcher als Anschlagstück (4) ausgebildet ist, das angetrieben bis zum Anschlagen an dem der Druckkammer abgewandten Ende des Arbeitskolbens beschleunigbar ist, wobei das Volumen der Druckkammer (1) größer ist als das Verdrängungsvolumen der von dem Arbeitskolben (3, 8) bei dessen Arbeitshub verdrängten Flüssigkeit, dadurch gekennzeichnet, daß von dem Anschlagstück (4) ein elastischer Stoß auf den Arbeitskolben ausübbar ist und das Anschlagstück (4) mit der Stoßübertragung auf den Arbeitskolben antriebslos ist.

2. Ejektionsgerät nach Anspruch 1, dadurch gekennzeichnet, daß das Anschlagstück als insbesondere zu dem Arbeitskolben (3, 8) koaxialer Antriebskolben (4) ausgebildet ist, der in einem Antriebsrohr (12) angetrieben beschleunigbar ist.

3. Ejektionsgerät nach Anspruch 1 oder 2, dadurch gekennzeichnet, daß das Anschlagstück (4) pneumatisch, hydraulisch, mechanisch, elektromagnetisch, elektrostriktiv, piezoelektrisch oder thermisch angetrieben ist.

4. Ejektionsgerät nach einem der Ansprüche 1 bis 3, dadurch gekennzeichnet, daß das Anschlagstück (4) für ein mehrfaches Anschlagen an dem Arbeitskolben (3, 8) periodisch hin- und hergehend antreibbar ist, wobei das Anschlagstück (4) und der Arbeitskolben (3, 8) selbsttätig rückstellbar sind.

5. Ejektionsgerät nach einem der Ansprüche 1 bis 4, dadurch gekennzeichnet, daß die Ejektionsöffnung (2) von einer Ventilvorrichtung beherrscht ist.

6. Ejektionsgerät nach Anspruch 5, dadurch gekennzeichnet, daß die Ventilvorrichtung auf eine vorbestimmte Drucksteigerung in der Druckkammer (1) unter Öffnen der Ejektionsöffnung (2) anspricht und druckabhängig rückstellbar ist.

7. Ejektionsgerät nach Anspruch 6, dadurch gekennzeichnet, daß die Ejektionsöffnung (2) in einer die Druckkammer (1) begrenzenden elastischen Membran (6) ausgebildet ist und in der Ruhelage der Membran vom freien Ende eines Verschlußstiftes (7) verschlossen wird.

8. Ejektionsgerät nach einem der Ansprüche 1 bis 7, dadurch gekennzeichnet, daß die Druckkammer (1) in mehrere Ejektionsöffnungen ausmündet.

9. Ejektionsgerät nach einem der Ansprüche 1 bis 8, dadurch gekennzeichnet, daß die Druckkammer (1) ein durch eine Druckabnahme in der Druckkammer öffnendes Einlaßventil aufweist.

10. Ejektionsgerät nach einem der Ansprüche 1 bis 9, dadurch gekennzeichnet, daß die Druckkammer in ihrem Aufnahmevolumen verstellbar ist.

11. Ejektionsgerät nach einem der Ansprüche 1 bis 10, gekennzeichnet durch die Ausbildung als endoskopisches Gerät, wobei der Arbeitskolben als elastische Stoßwellen auf die Flüssigkeit in der Druckkammer übertragende Sonde (8) ausgebildet ist.

12. Ejektionsgerät nach einem der Ansprüche 1 bis 11, dadurch gekennzeichnet, daß die Druckkammer (1) über einen engen Kanal mit einem Flüssigkeitsvorrat verbunden ist.

13. Ejektionsgerät nach Anspruch 12, dadurch gekennzeichnet, daß die Sonde an ihrem der Druckkammer (1) benachbarten Endabschnitt in einem Führungsstück (9) gleitend geführt ist, zwischen dem und der Sonde (8) ein enger Flüssigkeitsdurchtrittsspalt (10) ausgebildet ist, der proximal in einen entlang der Sonde (8) verlaufenden Flüssigkeitszuführkanal (11) mündet.

14. Ejektionsgerät nach einem der Ansprüche 1 bis 13, gekennzeichnet durch die Ausbildung als endoskopisches Gerät mit einem Endoskop-Katheter, in dem die Druckkammer als flüssigkeitsgefüllter Kanal ausgebildet ist.

15. Ejektionsgerät nach einem der Ansprüche 5 bis 14, gekennzeichnet durch die Ausbildung als nadelloses Impfgerät, wobei die Ventilvorrichtung in ihrem Öffnungsdruck verstellbar ist.

16. Ejektionsgerät nach einem der Ansprüche 1 bis 15, dadurch gekennzeichnet, daß das Verhältnis des Druckkammervolumens zu dem Verdrängungsvolumen des Antriebskolbens 5:1 bis insbesondere 20:1 beträgt.

## Claims

1. Ejection apparatus for the high pressure ejection of a liquid, or a particle containing liquid, having a pressure chamber (1) accomodating the liquid which pressure chamber (1) runs into an ejection opening (2) and is delimited by a working piston (3, 8) which can be displaced in the pressure chamber (1) by a drive, which drive is formed as an impact member which can be drivingly accelerated until it strikes on that end of the working piston which is remote of the pressure member, the volume of the pressure chamber (1) being larger than the displacement volume of the liquid displaced by the working piston (3, 8) during the working stroke thereof characterized in that an elastic impact on the working piston can be exerted by the impact member and the impact member is driveless upon the transmission of the impact to the working piston.

2. Ejection apparatus according to claim 1, characterised in that the impact member is formed as a driving piston (4) being coaxial particularly with respect to the working piston (3, 8) which driving piston (4) can be accelerated being driven in a driving pipe (5).

3. Ejection apparatus according to one of claims 1 or 2, characterised in that the impact member (4) is driven pneumatically, hydraulically, mechanically, electromagnetically, electrostrictively, piezo electrically or thermically.

4. Ejection apparatus according to one of claims 1 to 3, characterised in that the impact member (4) can be driven to periodically move forward and backward for a multiple striking on the driving piston (3, 8), wherein the impact member (4) and the working piston (3, 8) can be returned such that they are self-actuated.

5. Ejection apparatus according to one of the claims 1 to 4, characterised in that the ejection opening (2) is dominated by a valve device.

6. Ejection apparatus according to claim 5, characterised in that the valve device can react to a predetermined pressure increase in the pressure chamber (1) in that it opens the ejection opening (2) and can restore dependent on the pressure.

7. Ejection apparatus according to claim 6, characterised in that the ejection opening (2) is formed in a elastic membrane (6) which delimits the pressure chamber (1), and in the rest position of the membrane, is closed by the free end of a sealing pin (7).

8. Ejection apparatus according to one of claims 1 to 7, characterised in that the pressure chamber (1) runs into a plurality of injection openings.

9. Ejection apparatus according to one of claims 1 to 8, characterised in that the pressure chamber (1) comprises an inflow valve which opens as a result of a pressure decrease in the pressure chamber.

10. Ejection apparatus according to one of claims 1 to 9, characterised in that the pressure chamber (1) can be adjusted with respect to its accomodation volume.

11. Ejection apparatus according to one of claims 1 to 10, characterised by its construction as an endoscopic device, wherein the working piston is formed as a probe (8) transmitting elastic shock waves to the liquid in the pressure chamber.

12. Ejection apparatus according to one of claims 1 to 11, characterised in that the pressure chamber (1) is connected to a liquid reservoir via a narrow channel.

13. Ejection apparatus according to claim 12, characterised in that the probe, at its end section adjacent to the pressure chamber (1), is slidably guided in a guiding member (9), wherein between the guiding member (9) and the probe (8) a narrow liquid flow-through slit (10) is formed, which at the proximal end runs into a liquid supply channel (11) extending along the probe (8).

14. Ejection apparatus according to one of claims 1 to 13, characterised by its construction as an endoscopic device having an endoscope catheter, in which the pressure chamber is formed as a channel filled with liquid.

15. Ejection apparatus according to one of claims 5 to 14, characterised by its construction as an inoculation device without a needle, wherein the valve device can be adjusted with regard to its opening pressure.

16. Ejection apparatus according to one of claims 1 to 15, characterized in that the ratio of the volume of the pressure chamber to the displacement volume of the working piston is 5:1 to especially 20:1.

## Revendications

1. Dispositif d'éjection pour l'éjection à haute pression d'un liquide, ou d'un liquide contenant des particules, comprenant une chambre de pression (1) contenant le liquide, qui débouche à l'extérieur par une ouverture d'éjection (2) et qui est délimitée par un piston de travail (3, 8), qui est susceptible d'être déplacé dans la chambre de pression (1) par un moyen d'entraînement qui est réalisé sous la forme d'une pièce de percussion (4), laquelle est susceptible d'être accélérée jusqu'à la percussion sur l'extrémité du piston de travail opposée à la chambre de pression, le volume de la chambre de pression (1) étant supérieur à celui du volume de confinement du liquide réduit par le piston de travail (3, 8) au cours de la course de travail de ce dernier, caractérisé en ce que la pièce de percussion (4) est susceptible d'exercer un choc élastique sur le piston de travail et en ce que la pièce de percussion (4) n'est pas entraînée au cours de la transmission du choc au piston de travail.

2. Dispositif d'éjection selon la revendication 1, caractérisé en ce que la pièce de percussion est réalisée en particulier sous la forme d'un piston d'entraînement (4) coaxial au piston de travail (3, 8), et qui est susceptible d'être entraîné dans un tube d'entraînement (12).

3. Dispositif d'éjection selon la revendication 1 ou 2, caractérisé en ce que la pièce de percussion (4) est entraînée par voie pneumatique, hydraulique, mécanique, électromagnétique, électrostrictive, piézoélectrique ou thermique.

4. Dispositif d'éjection selon l'une des revendications 1 à 3, caractérisé en ce que la pièce de percussion (4) est susceptible d'être entraînée périodiquement en va et vient pour réaliser plusieurs percussions sur le piston de travail (3, 8), et en ce que la pièce de percussion (4) et le piston de travail (3, 8) sont susceptibles de revenir automatiquement.

5. Dispositif d'éjection selon l'une des revendications 1 à 4, caractérisé en ce que l'ouverture d'éjection (2) est contrôlée par un organe de valve.

6. Dispositif d'éjection selon la revendication 5, caractérisé en ce que l'organe de valve est sensible à une augmentation prédéterminée de la pression dans la chambre de pression (1) pour ouvrir l'ouverture d'éjection (2), et est susceptible de revenir en position en fonction de la pression (après une baisse de pression).

7. Dispositif d'éjection selon la revendication 6, caractérisé en ce que l'ouverture d'éjection (2) est réalisée dans une membrane élastique (6) délimitant la chambre de pression (1), et en position de repos de la membrane, est obturée par l'extrémité libre d'une broche de fermeture (7).

8. Dispositif d'éjection selon l'une des revendications 1 à 7, caractérisé en ce que la chambre de pression (1) débouche dans plusieurs ouvertures d'éjection.

9. Dispositif d'éjection selon l'une des revendications 1 à 8, caractérisé en ce que la chambre de pression (1) comporte une valve d'admission s'ouvrant par prélèvement de pression dans la chambre de pression.

10. Dispositif d'éjection selon l'une des revendications 1 à 9, caractérisé en ce que la chambre de pression présente un volume de réception réglable.

11. Dispositif d'éjection selon l'une des revendications 1 à 10, caractérisé par le fait qu'il est réalisé comme un appareil endoscopique, le piston de travail étant réalisé comme une sonde (8) transmettant au liquide dans la chambre de pression des ondes de choc élastiques.

12. Dispositif d'éjection selon l'une des revendications 1 à 11, caractérisé en ce que la chambre de pression (1) est reliée à un réservoir de liquide par un canal étroit.

13. Dispositif d'éjection selon la revendication 12, caractérisé en ce que la sonde est guidée de façon glissante dans une pièce de guidage (9) à son tronçon d'extrémité proche de la chambre de pression, en ce que, entre la pièce de guidage et la sonde (8), est formé un étroit intervalle (10) de passage de liquide qui débouche de façon proximale dans un canal (11) d'alimentation en liquide courant le long de la sonde (8).

14. Dispositif d'éjection selon l'une des revendications 1 à 13, caractérisé par le fait qu'il est réalisé comme un appareil endoscopique avec un cathéter d'endoscope, dans lequel la chambre de pression est réalisée sous la forme d'un canal rempli de liquide.

15. Dispositif d'éjection selon l'une des revendications 5 à 14, caractérisé par le fait qu'il est réalisé comme un appareil d'injection sans aiguille, l'organe de valve étant réglable en ce qui concerne sa pression d'ouverture.

16. Dispositif d'éjection selon l'une des revendications 1 à 15, caractérisé en ce que le rapport entre le volume de la chambre de pression et le volume de compression du piston d'entraînement est compris entre 5:1 jusqu'à en particulier 20:1.
